# EUROPEAN PATENT APPLICATION

(11) **EP 4 151 189 A1**
(43) Date of publication of application: **22.03.2023**
(21) Application number: 21198038.8
(22) Date of filing: 21.09.2021
(51) Int. Cl.: A61F 11/14, H04R 1/10

(54) **HEARING PROTECTION DEVICE WITH HAPTIC SENSING AND METHOD OF OPERATING A HEARING PROTECTION DEVICE**

(71) Applicant: 3M Innovative Properties Company, Saint Paul, MN 55133-3427 (US)
(72) Inventor: Nilsson, Jonas, 33133 Värnamo (SE); Hjort, Patrick, 33142 Värnamo (SE)
(74) Representative: Vollmers, Hans-Gerd

(57) **Abstract**

A hearing protection device (10, 10') comprises at least one earmuff (12, 14) or earplug (12', 14'), optionally a headband (18, 18') for carrying the at least one earmuff (12, 14) or earplug (12', 14'), electronic equipment (13b, 13c, 13d), sound-absorbing material and a haptic sensing unit (11, 11a, 11b) to sense a touch pattern occurring on at least a first portion of the hearing protection device (10, 10'), optionally of the at least one earmuff (12, 14) or earplug (12', 14') and/or of the headband (18, 18'). A method of operating a hearing protection device (10, 10') comprises the steps of providing a haptic sensing unit (11, 11a, 11b) having a haptic sensor (146a, 146b, 146c) configured to sense a touch pattern occurring on at least a first portion of the hearing protection device (10, 10'), optionally of the at least one earmuff (12, 14) or earplug (12', 14') and/or of the headband (18, 18'); executing a command when the haptic sensor senses (146a, 146b, 146c) a touch pattern occurring on at least a first portion of the hearing protection device (10, 10'), optionally at least one earmuff (12, 14) or earplug (12', 14') and/or of the headband (18, 18'); and triggering a particular state for the hearing protection device (10, 10') based on the touch pattern sensed, wherein the it provides for a button-less hearing protection device (10, 10') not requiring space and holes in the outer shell. Just tapping on a portion of the hearing protection device (10, 10') will initiate operational switching thereof, i. e.- upon sensing of a touch pattern, a command is executed.

## Description

The present disclosure relates to a hearing protection device with haptic sensing. The present disclosure also relates to a method of operating a hearing protection device.

Hearing protectors as personal protection equipment (PPE) are typically used in noisy environments for protecting a wearer's hearing from noise at potentially harmful noise levels. Typically, hearing protection devices have two muffs or caps which cover the ears of the wearer and which are connected to one another by a headband. Each cup further typically is formed by a rigid shell that is furnished with a noise dampening material, for example a foamed material. Alternatively, the hearing protection device may- instead of two earmuffs - comprise two earplugs, which may be connected by a headband.

There is a general desire to make hearing protection devices user-friendly, in particular to encourage persons that are in noisy environments for longer times to actually wear the protectors. Hearing protection devices have been developed and refined over time and may include electrical or electronic functions improving the protection and the comfort, respectively, of the wearers. Such hearing protection devices may include a noise cancelling function or other electronic functions such as communication with others over the hearing protection device and so on. These functions may require adjustments, which were typically done by the user pushing buttons on such devices, e. g. by hand. This may become difficult or even problematic if the work of the user requires both hands or if the user wears other personal protective equipment such as protective gloves so that operating the buttons may be difficult or impossible. One way to improve the adjustment may be to implement a voice recognition system into the hearing protection device so that voice commands by the user can adjust the functionality. However, such voice recognition systems may increase the costs of a hearing protection device and voice commands may not sufficiently be used particularly in noisy environments.

Therefore, a need exists to improve the control of a hearing protection device in situations when it is difficult to push control buttons. It is therefore an object of the present disclosure to improve the control of a hearing protection device.

In one aspect, the present disclosure relates to a hearing protection device comprising at least one earmuff or earplug, optionally a headband for carrying the at least one earmuff or earplug. The hearing protection device comprises electronic equipment, sound-absorbing material and a haptic sensing unit to sense a touch pattern occurring on at least a first portion of the hearing protection device, preferably of the at least one earmuff or earplug and/or of the headband. Such a hearing protection device is advantageous as it provides for a button-less hearing protection device. It allows for more signal combinations and no hole in outer shell and less space therein is required compared to buttons mounted in outer shell. Moreover, such a hearing protection device is easy to operate even if the user wears personal protective equipment such as protective gloves, which may make it cumbersome to operate buttons. Just tapping on a portion of the hearing protection device will initiate switching thereof. Thus, the present disclosure provides for a hearing protection device that comprises a haptic sensing unit that is configured to sense touch patterns. These touch patterns provide an efficient and rapid way for a user wearing the hearing protection device to access various features of the hearing protection device in a straightforward manner and without having to remove PPE, such as gloves.

In a second aspect, the present disclosure relates to a method of switching a hearing protection device from a first mode into a second mode. The hearing protection device comprises at least one earmuff or earplug, optionally a headband for carrying the at least one earmuff or earplug. The hearing protection device further comprises electronic equipment, sound-absorbing material and a haptic sensing unit to sense a touch pattern occurring on at least a first portion of the hearing protection device, preferably of the at least one earmuff or earplug and/or of the headband. The method comprises the steps of: providing a haptic sensing unit having a haptic sensor configured to sense a touch pattern occurring on at least a first portion of the hearing protection device, preferably of the at least one earmuff or earplug and/or of the headband; executing a command when the haptic sensor senses a touch pattern occurring on at least a first portion of the hearing protection device, preferably of the at least one earmuff or earplug and/or of the headband; and triggering a particular state for the hearing protection device based on the touch pattern sensed. The command optionally includes one or more of the following commands: Environmental sound on/off/volume, Connected device on/off/settings, Open/close microphone transmission, Switch between connected devices, Battery status, Connection to external device status, Wireless pairing, Take/quit incoming calls. It is understood by the skilled person that such a touch pattern could be used on hearing protection devices for different functions other than the ones listed above. The commands optionally comprise predefined and/or user definable commands. Such a method of operating a hearing protection device is advantageous as it provides for a button-less switching of the hearing protection device. It allows for more signal combinations and no hole in outer shell and less space therein is required compared to buttons mounted in outer shell. Moreover, such a method is easy to execute even if the user wears personal protective equipment such as protective gloves, which may make it cumbersome to operate buttons. Just tapping on a portion of the hearing protection device will initiate operational switching thereof.

A hearing protection device (HPD) within the meaning of the present disclosure is a personal protective equipment that comprises an ear protection worn in or over the ears while being exposed to hazardous or irritating ambient sounds or noise. An HPD helps to prevent noise-induced hearing impairment or even loss. HPD's may typically comprise two earmuffs or earplugs, although HPD's with one earmuff or earplug are conceivable. To achieve the protection, HPD's typically comprise active and/or passive noise reduction of cancellation means as described above. HPD's may typically also comprise a headband for connecting the two earmuffs or ear plugs and to keep these in position when the HPD is worn by a worker or user. The earmuffs of the HPD may be provided in the shape of a cup. Each cup further typically is formed by a rigid shell that is furnished with a sound-absorbing, acoustic damping or noise dampening material, for example a foamed material. Each cup has an ear-facing or skin-facing side, which - in use - faces towards the ear or skin of a user and a closed side, which faces away from the user's ear or skin. Earmuffs or earplugs typically comprise a cushion contacting the ear or skin of a user. Typically, materials for the cushion comprise a porous or foamed material. Materials suitable for providing sound-absorption may include porous or foamed materials , for example an EPDM (ethylene propylene diene monomer rubber) foam or PU (Polyurethane). The foamed material may include open pored or closed pored materials. Alternatively, the materials comprise a non-porous or foam-free material, e. g. silicone material of a theoretical density of between 1.1 g/cm³ and 1.2 g/cm³. Cushions of earmuffs may be provided as on-ear or over-ear cushions. The material and the structure of the cushion typically provides for resilient properties, in particular to allow an over-ear cushion to adapt tightly and sealingly with a user's or wearer's skin. It is also conceivable to include rubber as material for the cushion. The cushion may be cut-off or molded to achieve the desired size and shape.

Haptic within the meaning of the present disclosure is understood as kinesthetic communication or 3D touch and it includes any technology creating an experience of touch by applying forces, vibrations or motion by the user. Haptic relates to tactile, pertaining to the sense of touch. Different to pushing buttons for switching functions of a hearing protection device, haptic sensing does not involve acting a mechanical switch for switching these functions. Furthermore, typically mechanical switches are operated like on or off, whereas haptic switching may include frequency, intensity, repetition and/or touch patterns of the user tapping on the haptic sensing unit.

Commands within the meaning of the present disclosure may comprise predefined or prestored commands, which are pre-programmed into the hearing protection device, as well as user definable commands, which may be set up by a user. The latter represents a learning device, i. e. specific commands may be set up and programmed into the hearing protection device. The predefined commands may be considered as standard commands, whereas the user definable commands may be considered as free-definable commands.

In one embodiment, the electronic components of the hearing protector may be selected from an active communication unit, an active noise cancellation unit and/or other switchable components. Such components are advantageous as these provide for specific functionality required by the user of a hearing protection device and/or provide protection or comfort to the user of the hearing protection device.

In another embodiment, the haptic sensing unit of the hearing protection device comprises at least one haptic sensor, wherein the at least one haptic sensor optionally comprises at least one of an accelerometer, a transducer, and a touch sensor. An exemplary haptic sensor may comprise a transducer (e. g., a surface acoustic wave sensor), an accelerometer, an infrared sensor, and/or a resistive or capacitive touch sensor. Such a haptic sensor is advantageous as it provides for a reliable, compact and cost-efficient way of detecting haptic input/tapping on a portion of the hearing protection device.

In one embodiment, the haptic sensing unit of the hearing protection device may have software stored internally in, for example, a memory. Such a software may include characteristics of touch patterns, e. g. touch force, repetitions, or user behaviour. Such software is beneficial because user commands can easily be associated with switching of different functions of the hearing protection device for automatic or manual calibration.

In some embodiments, the one or more sensors of the hearing protection device can be coupled via hard wire to the haptic sensing unit. Such an arrangement is useful because hard wiring provides for a reliable connection to the sensor. Alternatively, the sensors may be wirelessly coupled to the haptic sensing unit, which provides for less wiring and more flexibility.

In another embodiment, a haptic sensor can comprise an accelerometer disposed within another portion of the hearing protection device. It may be beneficial to arrange the haptic sensor in that way to provide easier access for the user tapping onto the sensor.

Alternatively, the haptic sensing unit may comprise a haptic sensor disposed at or near any of the following locations: wearable gear, for example a wristband, or a control unit that clips onto clothes. In other words, the haptic sensor of the haptic sensing unit may be arranged spaced apart from the hearing protection device. This may provide for more freedom of usage and for a greater flexibility and/or comfort of the user.

In one embodiment, the haptic sensor unit of the hearing protection device is programmed to execute a command. For example, if a particular touch pattern is sensed, the haptic sensor unit is configured to execute a particular command based on the particular touch pattern sensed. As noted above, a touch pattern may comprise a single touch, a multiple touch (e. g., a rapid two-touch or three-touch pattern, etc.), or a series of single or multiple touches within a particular time period, or a type of touch, such as a light tap or a hard tap. As such, the haptic sensor unit may be programmed to execute multiple different commands based on any number of different touch patterns sensed. The commands optionally comprise predefined commands and/or user definable commands For example, the user-definable command may be one or more of the following commands: Radio on/off; Noise cancelling on/off; Feature on/off; Take/quit incoming calls; Accessory (on/off/control); Environmental sound on/off/volume; Connected device on/off/settings; Open/close microphone transmission; Switch between connected devices; Battery status; Connection to external device status; Wireless pairing. Such a hearing protection device is beneficial as the execution of such commands represents an easy and reliable way to control functions and status of a hearing protection device.

In some embodiments, the hearing protection device may further include control electronics disposed on a printed circuit board and electronic circuitry for switching the functions of the hearing protection device. The printed circuit board may have controls, in particular push buttons, by which the hearing protection device can be operated by a user. For example, the hearing protection device comprises a button for activating the automatic switching between the one mode and another mode. Other buttons may be provided, for example one or more buttons for switching power on or off, for switching the haptic sensing function on or off, for setting up specific modes of the haptic sensing such as quick functions. The buttons may also be arranged independent of control electronics. Such buttons in addition to the haptic sensors may provide for reliable switching of basic or important functions of the hearing protection device. Furthermore, buttons present in addition to haptic sensing may provide for some redundancy in case that haptic sensing does not work optimal, e. g. if the user wears gloves which may hinder the haptic input by the user.

In still another embodiment, the haptic sensing unit of the hearing protection device is programmed to execute a command, wherein the command optionally includes one or more of the following commands: Radio on/off, Noise cancelling on/off, Feature on/off; Take/quit incoming calls; Accessory (on/off/control); Environmental sound on/off/volume, Connected device on/off/settings, Open/close microphone transmission, Switch between connected devices, Battery status, Connection to external device status, Wireless pairing. The commands optionally comprise predefined commands and/or user definable commands. The hearing protection device may comprise standard or predefined commands. In addition thereto, the hearing protection device may be configured such that user-definable commands can be set-up and executed. It is also conceivable to define a command directly accessible through a specific touch pattern, e. g. an emergency command or the like. Such a haptic sensor unit is beneficial as the user may operate it in an easy and reliable way. The definition of user-definable or user-unique commands, which represents a learning function, may increase the user-friendliness of the hearing protection device. The user-definable commands provide for a convenient and reliable use of the hearing protection device, in particular the definition of an emergency command would help to initiate the required action without going through several menus or sub-menus.

In another embodiment, the hearing protection device may further have indications or labels for marking any controls (for example similar to the indication or labelling of push buttons) for operating the hearing protection device and its components, respectively. Furthermore, the indications or labels may have instructions for the user, for example, instruction about an adjustment of a switch function for a particular modus or application.

This is beneficial because such indications or labels help the user when operating the hearing protection device.

In yet another embodiment, the haptic sensor unit of the hearing protection device comprises a plurality of haptic sensors, wherein each sensor is disposed in a different portion of the at least one earmuff or earplug, optionally of the headband, wherein at least one haptic sensor optionally comprises at least one of an accelerometer, a transducer, and a touch sensor. Such a haptic sensor unit is useful as many different operations may be detected thereby, in particular with the plurality of haptic sensors, which may help to detect tapping of the user from different directions. Also, the detection of the haptic input may be improved due to multiple sensors used.

In a further embodiment, the hearing protection device comprises a first haptic sensing unit and a second haptic sensing unit, wherein the first haptic sensing unit comprises a first haptic sensor that senses a first touch pattern performed on a first portion of the at least one earmuff or earplug, optionally of the headband and the second haptic sensor that senses a second touch pattern performed on a second portion of the at least one earmuff or earplug, optionally of the headband, wherein the first and second portions are differently located on the hearing protection device. Such a first and second sensing unit are beneficial because the first and second sensing units could be split up to control different devices, for example the first sensing unit controls internal headset functions while the second sensing unit controls accessories. Furthermore, the first sensing unit could be used for shortcuts and user defined functions while the second sensing unit is used for browsing through command menus in detail.

In a certain further embodiment, the touch pattern of the hearing protection device includes a double touch and optionally a triple touch. Such a touch pattern may, for example, comprise touches of the user onto the hearing protection device detected by the haptic sensing unit comprising a certain number of touches each interrupted by certain time intervals. The detected touch pattern represents a command the user would like to give to the hearing protection device for switching of the electronic equipment embedded in the hearing protection device. The advantage of such touch patterns is that it is easy for the user to memorize these and to apply these onto the hearing protection device when inputting of a command is necessary. Also, specific touch patterns, e. g. a double touch or a double tapping, may be used to unlock the hearing protection device, so that further touches are recognized thereafter, e. g. single tapping. The risk of accidentally initiating the wrong command may be minimized thereby.

In certain further embodiments, the hearing protection device operates in a first mode when the haptic sensing unit senses a predetermined touch pattern such that the hearing protection device is switched into a second mode. This is beneficial because the hearing protection device with its electronic components can easily and reliably be switched from one mode to another thereby. Also, an accidentally wrong switching may be avoided or minimized in particular due to the use of a predetermined touch pattern for switching the modes, i. e. single tapping may not switch to another mode.

In certain further embodiments, the at least one haptic sensor of the hearing protection device is mounted on a portion of the at least one earmuff or earplug, optionally of the headband. Mounting the haptic sensor in that way represents an easy, reliable and compact design for operating the hearing protection device. It may be conceivable to arrange a haptic sensor on a microphone or microphone boom present on the hearing protection device. Easy access to the haptic sensor may be provided for the user thereby.

In certain further embodiments, the at least one haptic sensor unit of the hearing protection device is configured to distinguish between a first touch pattern executed on a first portion of the at least one earmuff or earplug, optionally of the headband and a second touch pattern executed on a second portion of the at least one earmuff or earplug, optionally of the headband. Such an arrangement of the haptic sensors provides more freedom to the user for inputting the command by tapping on different portions of the hearing protection device for switching different functions, which will be recognized by the haptic sensor in that way.

In a certain further embodiment, the first portion of the hearing protection device is located at the first earmuff or earplug and wherein the second portion of the hearing protection device is located at the second earmuff or earplug. The user is provided with an easy choice of selecting the way to input commands by tapping either in the first or second earmuff or earplug. A greater variety of commands may also be provided thereby as well as more quick commands without resulting in too complicated tapping patterns.

In certain further embodiments, the at least one earmuff or earplug of the hearing protection device comprises an outer shell, which is - in use - oriented away from the user's skin, wherein the outer shell comprise an x-, y- and z-axis and wherein haptic sensors are configured and arranged such sensing of a touch pattern is achieved in two directions for the x, y-axis and z-axis. Such an arrangement represents a structured way with which the user can input commands for switching of the hearing protection device and its electronic components. In particular, defining x-, y- and z-axes may make it easier for the user to input the commands as the user can easily find these directions. It is noted that in practice, there might only be one direction present for the z-axis as - in use - the user's head may be in the way for tapping on the outer shell from one direction along the z-axis.

In a certain further embodiment, the x-axis of the outer shell of the hearing protection device is oriented is aligned along a frontal plane, the y-axis is aligned along a sagittal plane and the z-axis is aligned along a transversal plane. Such an orientation has the advantage that an easy and reliable way is provided to the user for inputting the commands by tapping in directions along the above-mentioned axes. However, it is conceivable that a hearing protection device is worn in a slightly tilted way. It is understood that the orientation of the axes needs to be adapted thereto.

In certain further embodiments, the hearing protection device may comprise a three-axes accelerometer. The three axes accelerometer may be configured and arranged to detect inputs from six different directions such as along the x-axis, the y-axis and the z-axis and for each of the axes in a positive and negative direction. The advantage of such an accelerometer is that only one accelerometer is needed for the detection of the haptic input which would be a cost-efficient solution. It is also conceivable that the three-axes accelerometer is configured and arranged such that not only input along one of the axes (x-axis, y-axis or z-axis) is detected, but also input along a direction tilted to one of these axes. For example, the accelerometer may be configured to calculate the input detected and determine the exact direction.

In a certain further embodiment, the outer shell of the hearing protection device comprises two haptic sensors arranged on the outer shell in the frontal and the sagittal plane and one haptic sensor arranged on the outer shell in the transversal plane. Such an arrangement of haptic sensors has the advantage that an easy and reliable way is provided to the user for inputting the commands by tapping in directions along the above-mentioned axes. It is conceivable that the haptic sensors of the hearing protection device are configured and arranged such that tapping on different locations, i. e. following detection of the input by different sensors, may result in switching different functions of the hearing protection device. For example, tapping on top of the earmuff or earplug may increase the volume of the loudspeaker present in the earmuff or earplug, whereas tapping on a lower part may result in decreasing the volume. Other set up of locations, sensors and functions is conceivable. The advantage of such a set is that an easy and reliable way of switching is provided to the user, which may also be used in an intuitive way.

The present disclosure is drawn to a hearing protection device with haptic sensing capabilities. In particular, the hearing protection device comprises a haptic sensing unit that is configured to sense touch patterns, be it a single touch, a multiple touch, a series of single/multiple touches, or a heavy or light touch, that allow a user wearing the hearing protection device to change operational states and/or access one or more features of the hearing protection device in a straightforward and rapid manner. The type of quick access provided by the hearing protection device of the present disclosure can be extremely convenient and/or important for the user, especially in some noisy environments. These environments may make finding and actuating a conventional button, dial, switch or other mechanical actuation device cumbersome or difficult, and may require precision manipulation by the user and may even necessitate removing equipment (e. g., removing protective gloves). By implementing haptic sensing locations on one or more parts of the hearing protection device, the requirement for such precision is reduced and the system controllability can be improved. For example, in conventional hearing protection device, entering a particular mode, such as a Setting mode, Active mode, Input mode, Unlocked mode or Receiving mode, requires accessing and manually activating an external button on the hearing protection device or accessing the user interface on the hearing protection device. This conventional approach can complicate hearing protection device design and safety. In addition, accessing such a Setting mode, Active mode, Input mode, Unlocked mode or Receiving mode activation button may be difficult in certain noisy environments, in particular if the user wears personal protective equipment, for example protective gloves which may reduce the user's hands sensitivity.

With the hearing protection device of the present disclosure, a user of a hearing protection device may instead tap anywhere on the hearing protection device to switch to/from Setting mode, Active mode, Input mode, Unlocked mode or Receiving mode.

Before describing in detail exemplary embodiments that are in accordance with the disclosure, it is noted that components have been represented where appropriate by conventional symbols in drawings, showing only those specific details that are pertinent to understanding the embodiments of the disclosure so as not to obscure the disclosure with details that will be readily apparent to those of ordinary skill in the art having the benefit of the description herein.

As used herein, relational terms, such as "first," "second," "top" and "bottom," and the like, may be used solely to distinguish one entity or element from another entity or element without necessarily requiring or implying any physical or logical relationship or order between such entities or elements. The terminology used herein is for the purpose of describing particular embodiments only and is not intended to be limiting of the concepts described herein. As used herein, the singular forms "a", "an" and "the" are intended to include the plural forms as well, unless the context clearly indicates otherwise. It will be further understood that the terms "comprises," "comprising," "includes" and/or "including" when used herein, specify the presence of stated features, integers, steps, operations, elements, and/or components, but do not preclude the presence or addition of one or more other features, integers, steps, operations, elements, components, and/or groups thereof.

Unless otherwise defined, all terms (including technical and scientific terms) used herein have the same meaning as commonly understood by one of ordinary skill in the art to which this disclosure belongs. It will be further understood that terms used herein should be interpreted as having a meaning that is consistent with their meaning in the context of this specification and the relevant art and will not be interpreted in an idealized or overly formal sense unless expressly so defined herein.

It is generally understood that that the contemplated modes of operation are similar to how hearing protection devices are normally being operated and controlled, except that haptic input is used instead of buttons for switching functions of the hearing protection device. The haptic input may also be used to control other device, for example control calls and media streaming from a mobile phone.

The invention was described in various embodiments above. It is understood by a person skilled in the art, that one of, several of or all the above-mentioned embodiments can be combined with each other.

The invention will now be described in more detail with reference to the following Figures exemplifying particular embodiments of the invention:

### BRIEF DESCRIPTION OF THE DRAWINGS

Figs. 1A and 1B are perspective views of an embodiment of the hearing protection device according to the present disclosure;
Fig. 2 is a schematic functional view of another embodiment of the hearing protection device according to the present disclosure;
Fig. 3 is a schematic view of a haptic sensing unit of the hearing protection device according to the present disclosure;
Fig. 4 is a flowchart of a process to change the operational state of the hearing protection device according to the present disclosure;
Fig. 5A is a schematic perspective view of the hearing protection device and axes oriented thereon;
Fig. 5B is a schematic perspective view of the axes as illustrated in Fig. 5A and their orientation and
Fig. 5C is a schematic perspective view of the x- and z-axes as illustrated in Fig. 5B.

### DETAILED DESCRIPTION

Referring now to the drawing figures in which like reference designations refer to like elements, an embodiment of a hearing protection device constructed in accordance with the principles of the present disclosure is shown in the figures and generally designated as 10 or 10'.

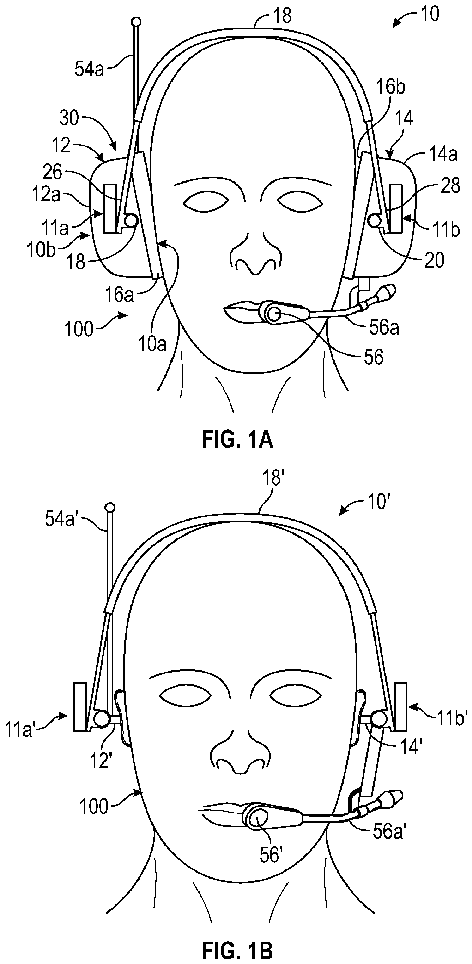

The hearing protection device 10 shown in Figs. 1A and 1B generally includes two earmuffs 12, 14 or earplugs 12', 14' and a headband 18, 18' supporting the earmuffs 12, 14 or earplugs 12', 14' when the hearing protection device is worn by a user 100 on the user's head.

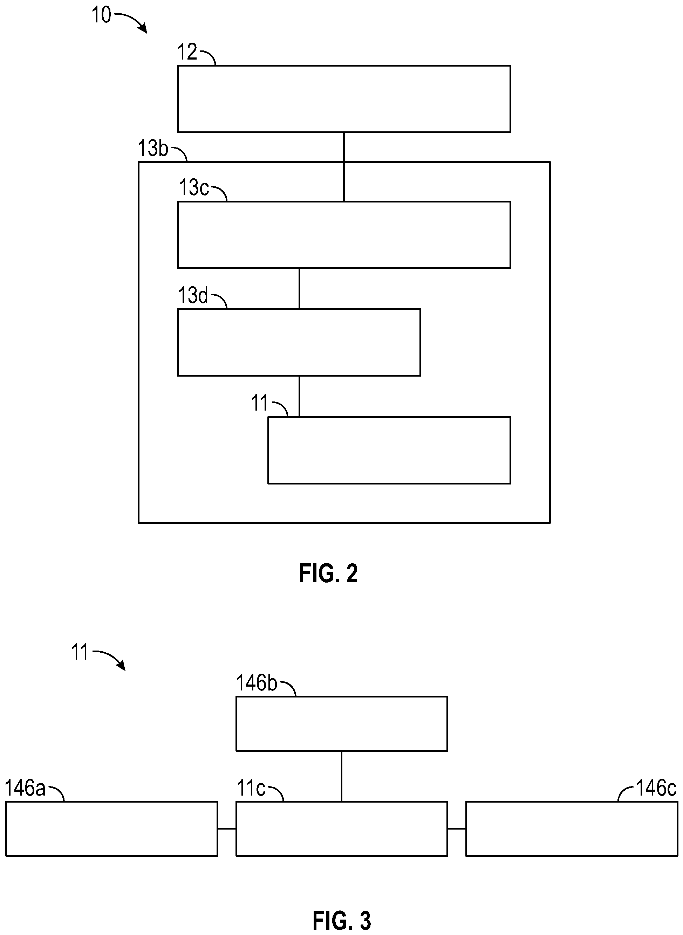

Fig. 1A further shows in a perspective view the hearing protection device 10 having a microphone 56 carried by a microphone boom 56a extending from one of the earmuffs 14 and an antenna 54a extending from one earmuff 12. The microphone 56 and the antenna 54a may be part of an active communication system of the hearing protection device 10. Although the microphone boom 56a extends from the earmuff 14 and the antenna 54a extends from one earmuff 12, it is understood that other constructions are conceivable as well, e. g. that the microphone boom 56a and/or the antenna 54a extends from the other earmuff 12, 14 or from the headband 18. The hearing protection device 10 further comprises the haptic sensing units 11a, 11b. Details of a haptic sensing unit 11 including a control unit 11c and one or more haptic sensors 146a, 146b, 146 c (not shown here) are illustrated in Fig. 3 below.

Fig. 1A further illustrates more details of the hearing protection device 10 worn by the user 100 and showing further details of the hearing protection device 10. The hearing protection device 10 comprises earmuffs 12, 14 having outer shells 12a, 14a and earmuff connections 18, 20 extending therefrom which are connected to the headband 18 through the headband connections 26, 28 arranged on the ends of the headband 18 engaging the earmuff connections 18, 20. Earmuffs 12, 14 further each comprise a cushion 16a, 16b mounted on the outer shells 12a, 14a of the earmuffs 12, 14 at a side facing - in use as shown here - towards the skin of the user 100. The skin-facing side is indicated with 10a, whereas the side facing - in use - away from the skin of the user 100 is indicated with 10b. The hearing protection device 10 comprises a haptic sensing unit arranged on each of the earmuffs 12, 14 as illustrated with 11a, 11b.

Fig. 1B shows an embodiment being similar to the one shown in Fig. 1A except that the hearing protection device 10' comprises earplugs 12', 14' instead of the earmuffs 12, 14 of the hearing protection device 10 as shown in Fig. 1A. Similar to Fig. 1A, Fig. 1B shows haptic sensing units 11a', 11b' attached to the hearing protection device 10'. Details of a haptic sensing unit 11 including a control unit 11c and one or more haptic sensors 146a, 146b, 146 c (not shown here) are illustrated in Fig. 3 below. Fig. 1B shows the hearing protection device worn by a user 100. The hearing protection device 10' of Fig. 1B also has a headband 18' supporting the earplugs 12', 14'. An antenna 54a' and a microphone 56 supported by a microphone boom 56a', which all may be part of an active communication device, are arranged at the earplugs 12', 14'. Although the microphone boom 56a' extends from the earplug 14' and the antenna extends from one earplug 12', it is understood that other constructions are conceivable as well, e. g. that the microphone boom 56a' and/or the antenna 54a' extends from the other earplug 12', 14' or from the headband 18.

As will be apparent to one of skill in the art given the present description, embodiments of the invention are not limited to the specific design of the hearing protection device 10, 10' shown in Figs. 1A and 1B, as the haptic sensing unit described herein may be incorporated into alternative hearing protection device configurations.

Fig. 2 provides a schematic view of portions of the hearing protection device 10 shown in Fig. 1A. The hearing protection device 10 may include a controller or controller electronics 13b. As exemplarily shown, the controller or controller electronics 13b is connected to the earmuff 12 of the hearing protection device 10. It is understood that the same arrangement is conceivable for the earmuff 14 (not shown). The controller or controller electronics 13b may include a controller and detection unit 13c and a processor 13d. In addition, in accordance with embodiments of the invention, the haptic sensing unit 11 may also be disposed on the controller electronics 13b of the hearing protection device 10. It is understood that the configuration as described here may also apply for a hearing protection device 10' with earplugs 12', 14'.

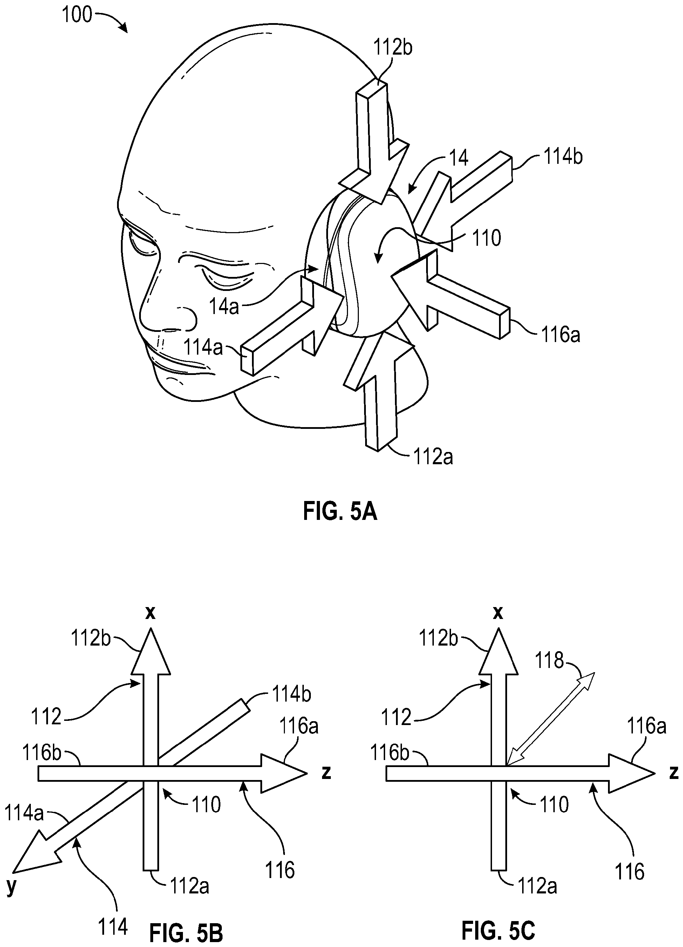

The haptic sensing unit 11 may comprise a control unit 11c and one or more haptic sensors, e. g., haptic sensors 146a, 146b, 146c as shown in FIG. 3, that may be disposed at one or more locations within hearing protection device 10, 10'. It is noted that two haptic sensing units 11a, 11b may be present at the hearing protection device 10, 10'. Each haptic sensor 146a, 146b, 146c may detect a touch pattern, such as a single or multiple touch, at a particular location on the hearing protection device 10, 10', such as the one of the earmuffs or earplugs 12, 14 and/or the headband 18, or other portions of the hearing protection device 10, 10', e. g. the antenna 54a and/or the microphone 56 or microphone boom 56a. In accordance with yet another embodiment of the present disclosure, for a haptic sensor 146a, 146b, 146c deployed to sense touch pattern on the hearing protection device 10, 10', one or more sensors 146a, 146b, 146c may be deployed to sense and differentiate between touch patterns made on different areas of the hearing protection device 10, 10' - for example, the earmuffs 12, 14 or earplugs 12', 14' and different locations thereon, the headband 18, the antenna 54a, 54a' and/or the microphone 56, 56' or microphone boom 56a, 56a'. It is noted that the earmuffs 12, 14 may comprise an outer shell 12a, 14a as, for example, illustrated in Fig. 1B for the earmuffs 12, 14. It is further noted that also earplugs 12', 14' each may comprise an outer shell (not shown in Fig. 1B). Different locations onto which tapping of a user 100 is conceivable as command input are shown and explained in Fig. 5 below. In addition, the one or more haptic sensors may be configured to distinguish between a touch pattern present on a first portion of the hearing protection device 10, 10' versus a touch pattern present on another portion of the hearing protection device 10, 10'.

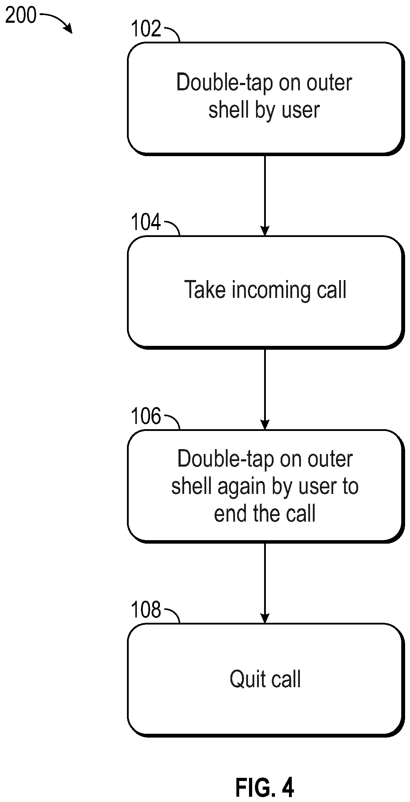

Fig. 4 is a flowchart of a process to change the operational state of a hearing protection device 10 in accordance with embodiments of the present disclosure. As can be seen, step 102 represents the user 100 double-tapping on a portion of the hearing protection device 10, e. g. the outer shell 12a, 14a of one of the earmuffs 12, 14 and thereby inputting a command into the haptic sensing unit 11 and the haptic sensor 146a, 146b, 146c (not shown here). The haptic sensing unit 11 with its control unit 11c will exemplarily switch the active communication unit in order to take an incoming call which is illustrated with step 104. After doing and upon finishing the call, the user 100 again is double tapping on a portion of the hearing protection device 10, e. g. again on the outer shell 12a, 14a of one of the earmuffs 12, 14 which is illustrated in step 106 to initiate the command of ending the call. The haptic sensing unit 11 with its haptic sensor 146a, 146b, 146c (not shown here) of hearing protection device 10 will initiate this command at the active communication unit of the hearing protection device 10 and quit the previously taken call which is indicated by step 108. It is noted that the above-described process is also conceivable for earplugs 12', 14' of the hearing protection device 10' (not illustrated here).

Fig. 5A is a schematic perspective view of the hearing protection device 10, which is only partially shown here, i. e. the outer shell 14a of the earmuff 14 with axes oriented thereon. As can be seen, the x-axis 112 is oriented vertically and along a frontal plane. The y- and z-axes 114, 116 are arranged horizontally and perpendicular to each other such that the y-axis 114 is oriented in a sagittal plane and the z-axis 116 is oriented in a transversal plane. The axes are oriented such that a zero point 110 is formed as illustrated in Fig. 5A. An illustration of the arrangement of x-, y- and z-axes 112, 114, 116 and the positive and negative scales 112a, 112b, 114a, 114b, 116a, 116b is provided in Fig. 5B and described below. The user 100 may tap onto the outer shell 14a of the earmuff 14 horizontally from the front or the rear, i. e. along the y-axis 114 in a positive (indicated with 114a) or negative scale (indicated with 114b) thereof seen from the zero point 110. Alternatively, the user 100 may also tap onto the outer shell 14a of the earmuff 14 vertically from above or below, i. e. along the x-axis in a positive (indicated with 112a) or negative scale (indicated with 112b) thereof seen from the zero point 110. In a third alternative, the user 100 may tap onto the outer shell 14a of the earmuff 14 horizontally towards the user's head, i. e. along the z-axis in a positive scale (indicated with 116a) seen from the zero point 110. There is no negative scale (indicated with 116b) of the z-axis available for tapping as the head of the user is in the way of tapping in the negative scale of the z-axis indicted with 116b seen from the zero point 110 in Fig. 5B. Such an arrangement provides for five possibilities of tapping directions onto the outer shell 14a of the earmuff 14. However, it may also be conceivable to detect tapping in a direction angled to one of the x-, y- or z-axes, i. e. in a tilted direction. This may, for example, be done by calculating it from the accelerometer data or by adding tilted accelerometers. Fig. 5C illustrates such a tilted arrangement of the haptic input. Fig. 5A shows the arrangement of the x-, y- and z-axes 112, 114, 116 for one earmuff 14 only. It is understood that both earmuffs 12, 14 may comprise such an arrangement. Although shown for the hearing protection device 10 with earmuffs 12, 14, such an arrangement with x-, y- and z-axes 112, 114, 116 may also be conceivable for the hearing protection device 10' with earplugs 12', 14', either on one or both earplugs 12', 14'.

Fig. 5B shows in a 3-dimensional illustration of the x-, y- and z-axes 112, 114, 116 as referred to under Fig. 5A. The hearing protection device 10 has been omitted in this view for the sake of simplification. The x-, y- and z-axes 112, 114, 116 form a cartesian coordinate system with the intercept point or zero point 110, i. e. each of the x-, y- and z-axes 112, 114, 116 is perpendicular to the two other axes. As can be seen, the x-axis 112 is oriented vertically and along a frontal plane. The y- and z-axes 114, 116 are arranged horizontally and perpendicular to each other such that the y-axis 114 is oriented in a sagittal plane and the z-axis 116 is oriented in a transversal plane. The axes 112, 114, 116 are oriented such that a zero point 110 is formed as illustrated in Fig. 5A.

Fig. 5C shows in a 2-dimensional illustration the x- and z-axes 112, 116 as illustrated in Fig. 5B. The y-axis 114, which is shown in Fig. 5B, has been omitted here for the sake of simplification. Fig. 5C shows a dotted line 118 to illustrate a direction for the haptic input not strictly aligned with one of the x- or z-axes 112, 116, i. e. direction 118 of the haptic input is tilted with respect to the x- and z-axes 112, 116. It is noted, although not shown here, that such a tilted arrangement for the haptic input may occur also between other axes, e. g. between the x- and y-axes 112, 114 or between the y- and z-axes 114, 116.

As would be apparent to one of skill in the art given the present description, alternative methods having different touch patterns, different switching functions, or combinations thereof may be also be implemented with the hearing protection device 10, 10'. In addition, the haptic sensors 146a, 146b, 146c of the haptic sensing unit 11, 11' may be disposed at different locations of the hearing protection device 10, 10', so that the user/wearer can switch a specific function, can activate a specific mode or can switch to another operational mode by contacting that portion of the hearing protection device 10, 10' with the appropriate touch pattern.

It should be understood that various aspects disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e. g., all described acts or events may not be necessary to carry out the techniques). In addition, while certain aspects of this disclosure are described as being performed by a single module or unit for purposes of clarity, it should be understood that the techniques of this disclosure may be performed by a combination of units or modules associated with, for example, a medical device.

In one or more embodiments, the described techniques may be implemented in hardware, software, firmware, or any combination thereof. If implemented in software, the functions may be stored as one or more instructions or code on a computer-readable medium and executed by a hardware-based processing unit. Computer-readable media may include non-transitory computer-readable media, which corresponds to a tangible medium such as data storage media (e. g., RAM, ROM, EEPROM, flash memory, or any other medium that may be used to store desired program code in the form of instructions or data structures and that can be accessed by a computer).

Instructions may be executed by one or more processors, such as one or more digital signal processors (DSPs), general purpose microprocessors, application specific integrated circuits (ASICs), field programmable logic arrays (FPGAs), or other equivalent integrated or discrete logic circuitry. Accordingly, the term "processor" as used herein may refer to any of the foregoing structure or any other physical structure suitable for implementation of the described techniques. Also, the techniques could be fully implemented in one or more circuits or logic elements.

## Claims

1. A hearing protection device (10, 10') comprising:
at least one earmuff (12, 14) or earplug (12', 14'), optionally a headband (18, 18') for carrying the at least one earmuff (12, 14) or earplug (12', 14');
electronic equipment (13b, 13c, 13d);
sound-absorbing material and
a haptic sensing unit (11, 11a, 11b) to sense a touch pattern occurring on at least a first portion of the hearing protection device (10, 10'), preferably of the at least one earmuff (12, 14) or earplug (12', 14') and/or of the headband (18, 18').

2. The hearing protector (10, 10') according to claim 1, wherein the electronic components (13b, 13c, 13d) may be selected from an active communication unit, an active noise cancellation unit and/or other switchable components.

3. The hearing protection device (10, 10') according to any one of claims 1 or 2, wherein the haptic sensing unit (11, 11a, 11b) comprises at least one haptic sensor (146a, 146b, 146c), wherein the at least one haptic sensor (146a, 146b, 146c) optionally comprises at least one of an accelerometer, a transducer, and a touch sensor.

4. The hearing protection device (10, 10') according to claim 3, wherein the haptic sensing unit (11, 11a, 11b) is programmed to execute a command, wherein the command optionally includes one or more of the following commands: Radio on/off, Noise cancelling on/off, Feature on/off; Take/quit incoming calls; Accessory (on/off/control), Environmental sound on/off/volume, Connected device on/off/settings, Open/close microphone transmission, Switch between connected devices, Battery status, Connection to external device status, Wireless pairing, wherein the commands optionally comprise predefined and/or user definable commands.

5. The hearing protection device (10, 10') according to any one of claims 1 to 4, wherein the haptic sensing unit (11, 11a, 11b) comprises a plurality of haptic sensors (146a, 146b, 146c), wherein each sensor (146a, 146b, 146c) is disposed in a different portion of the at least one earmuff (12, 14) or earplug (12', 14'), optionally of the headband (18, 18'), wherein at least one haptic sensor (146a, 146b, 146c) optionally comprises at least one of an accelerometer, a transducer, and a touch sensor.

6. The hearing protection device (10, 10') according to any one of claims 1 to 5 comprising a first haptic sensing unit (11a) and a second haptic sensing unit (11b), wherein the first haptic sensing unit (11a) comprises a first haptic sensor (146a, 146b, 146c) that senses a first touch pattern performed on a first portion of the at least one earmuff (12, 14) or earplug (12', 14'), optionally of the headband (18, 18') and the second haptic sensor (146a, 146b, 146c) that senses a second touch pattern performed on a second portion of the at least one earmuff (12, 14) or earplug (12', 14'), optionally of the headband (18, 18'), wherein the first and second portions are differently located on the hearing protection device (10, 10').

7. The hearing protection device (10, 10') to any one of claims 1 to 6, wherein the touch pattern includes a double touch and optionally a triple touch.

8. The hearing protection device (10, 10') according to any one of claims 1 to 7, wherein the hearing protection device (10, 10') operates in a first mode when the haptic sensing unit (11, 11a, 11b) senses a predetermined touch pattern such that the hearing protection device (10, 10') is switched into a second mode.

9. The hearing protection device (10, 10') according to any one of claims 3 to 8, wherein the at least one haptic sensor (146a, 146b, 146c) is mounted on a portion of the at least one earmuff (12, 14) or earplug (12', 14'), optionally of the headband (18, 18').

10. The hearing protection device (10, 10') according to any one of claims 3 to 9, wherein the at least one haptic sensor unit (11, 11a, 11b) is configured to distinguish between a first touch pattern executed on a first portion of the at least one earmuff (12, 14) or earplug (12', 14'), optionally of the headband (18, 18') and a second touch pattern executed on a second portion of the at least one earmuff (12, 14) or earplug (12', 14'), optionally of the headband (18, 18').

11. The hearing protection device (10, 10') according to claim 10, wherein the first portion is located at the first earmuff (12, 14) or earplug (12', 14') and wherein the second portion is located at the second earmuff (12, 14) or earplug (12', 14').

12. The hearing protection device (10, 10') according to any one of claims 5 to 11, wherein the at least one earmuff (12, 14) or earplug (12', 14') comprises an outer shell (12a, 14a), which is - in use - oriented away from the user's skin, wherein the outer shell (12a, 14a) comprises an x-, y- and z-axis (112, 114, 116) and wherein haptic sensors (146a, 146b, 146c) are configured and arranged such sensing of a touch pattern is achieved in two directions for the x, y-axis and z-axis (112, 114, 116).

13. The hearing protection device (10, 10') according to claim 12, wherein the x-axis (112) is aligned along a frontal plane, the y-axis (114) is aligned along a sagittal plane and the z-axis (116) is aligned along a transversal plane.

14. The hearing protection device (10, 10') according to claim 13, comprising a three-axes accelerometer configured and arranged to detect inputs from six different directions such as along the x-axis, the y-axis and the z-axis (112, 114, 116) and for each of the axes (112, 114, 116) in a positive and negative direction.

15. A method of switching a hearing protection device (10, 10') from a first mode into a second mode, the hearing protection device (10, 10') comprising at least one earmuff (12, 14) or earplug (12', 14'), optionally a headband (18, 18') for carrying the at least one earmuff (12, 14) or earplug (12', 14'), electronic equipment (13b, 13c, 13d), sound-absorbing material and a haptic sensing unit (11, 11a, 11b) to sense a touch pattern occurring on at least a first portion of the hearing protection device (10, 10'), preferably of the at least one earmuff (12, 14) or earplug (12', 14') and/or of the headband (18, 18'), the method comprising the steps of:
providing a haptic sensing unit (11, 11a, 11b) having a haptic sensor (146a, 146b, 146c) configured to sense a touch pattern occurring on at least a first portion of the hearing protection device (10, 10'), preferably of the at least one earmuff (12, 14) or earplug (12', 14') and/or of the headband (18, 18');
executing a command when the haptic sensor senses (146a, 146b, 146c) a touch pattern occurring on at least a first portion of the hearing protection device (10, 10'), preferably of the at least one earmuff (12, 14) or earplug (12', 14'), and/or of the headband (18, 18'); and
triggering a particular state for the hearing protection device (10, 10') based on the touch pattern sensed,
wherein the command optionally includes one or more of the following commands:
Environmental sound on/off/volume, Connected device on/off/settings, Open/close microphone transmission, Switch between connected devices, Battery status,
Connection to external device status, Wireless pairing, Take/quit incoming calls,
wherein the commands optionally comprise predefined and/or user definable commands.
